(19) Europäisches Patentamt / European Patent Office / Office européen des brevets

(11) **EP 3 194 619 B1**

(12) **EUROPEAN PATENT SPECIFICATION**

(45) Date of publication and mention
of the grant of the patent:
**06.03.2019 Bulletin 2019/10**

(21) Application number: **15775119.9**

(22) Date of filing: **17.09.2015**

(51) Int Cl.:
***C12Q 1/6827*** *(2018.01)*

(86) International application number:
**PCT/EP2015/071303**

(87) International publication number:
**WO 2016/042067 (24.03.2016 Gazette 2016/12)**

(54) **METHODS AND TOOLS FOR ANALYZING HYBRIDIZATION**

VERFAHREN UND WERKZEUGE ZUR ANALYSE VON HYBRIDISIERUNG

PROCÉDÉS ET OUTILS POUR L'ANALYSE D'HYBRIDATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2014 EP 14185208**

(43) Date of publication of application:
**26.07.2017 Bulletin 2017/30**

(73) Proprietor: **VITO NV**
**2400 Mol (BE)**

(72) Inventors:
• **HOOYBERGHS, Jef**
**2400 Mol (BE)**
• **JACOBS, An**
**2400 Mol (BE)**
• **WILLEMS, Hanny**
**2400 Mol (BE)**

(74) Representative: **V.O.**
**P.O. Box 87930**
**2508 DH Den Haag (NL)**

(56) References cited:
**EP-A2- 0 995 804        WO-A1-95/11995**
**WO-A1-2011/035801**

• **HOOYBERGHS J ET AL: "Hybridisation thermodynamic parameters allow accurate detection of point mutations with DNA microarrays", BIOSENSORS AND BIOELECTRONICS, ELSEVIER BV, NL, vol. 26, no. 4, 15 December 2010 (2010-12-15), pages 1692-1695, XP027546903, ISSN: 0956-5663 [retrieved on 2010-08-16]**
• **W. W. HADIWIKARTA ET AL: "Targeted resequencing of HIV variants by microarray thermodynamics", NUCLEIC ACIDS RESEARCH, vol. 41, no. 18, 8 August 2013 (2013-08-08), pages e173-e173, XP055172611, ISSN: 0305-1048, DOI: 10.1093/nar/gkt682**

**Description**

**FIELD OF THE INVENTION**

The present invention relates to methods for analyzing hybridization, more particularly to determine the presence or absence of specific polynucleotides such as mutated genes in a sample. Further provided herein are related kits and computer programs for analyzing hybridization.

**BACKGROUND OF THE INVENTION**

In targeted therapies used for treating cancer it is known that the efficacy across patient populations can be widely distributed. The knowledge on the underlying mechanisms is growing continuously and can often be translated into certain mutations of the cancer cell's DNA. Thus, an accurate identification of reliable mutation markers is crucial for optimizing treatment response.

However, such identification can be complicated because of the heterogeneity of the tissue material obtained from the patient. Indeed, the ratio of tumor versus non-tumor cells in clinical samples may vary and the mutations may be heterozygous or homozygous. Moreover, the set of known relevant biomarkers may change over time. Thus, reliable diagnostic test should be flexible towards these constraints.

In practice, the detection of DNA mutations is typically performed using polymerase chain reaction (PCR) and sequencing. The use of hybridization techniques like microarrays for the detection of DNA mutations is rather uncommon, even though this technology is mature, affordable, widely used and very flexible towards type and number of DNA sequences to target.

A remaining challenge with hybridization techniques is obtaining a clear quantitative interpretation of microarray data. WO2011/035801 describes a method for analyzing hybridization, involving the analysis of hybridization intensities for different probes as a function of hybridization free energy. Although the method allows for identifying which of a known set of mutants is present in a sample, there is still a need for improved methods for analyzing hybridization.

Hooyberghs et al. (Biosensors and Bioelectronics, 2010, 26: 1692-1694) relates to a microarray and hybridization-based method of detecting small concentrations in a mixture of mutant and wild type polynucleotides, based on the observation of a shift of cluster of probes with respect to a thermodynamic baseline (by plotting the hybridization intensity against the $\Delta\Delta G$), wherein hybridization intensities obtained for the mixture are compared to this thermodynamic baseline. However, this method is prone to errors due to concentration variations in the sample.

In EP0995804, a target polynucleotide and a reference polynucleotide are individually hybridized to two identical probe arrays, wherein the presence of a mutation in the target polynucleotide is determined by comparing the hybridization patterns obtained for the target polynucleotide and reference polynucleotide. WO9511995 discloses a method involving hybridizing a reference and a target sequence to identical arrays comprising a plurality of probes comprising several mismatch probes and determining whether the reference sequence is the same or different from the target sequence based on the relative specific binding to the probes.

There is thus still a need for methods and tools for the reliable identification and/or quantification of mutant polynucleotides in a sample.

**SUMMARY OF THE INVENTION**

The present invention relates to methods for analyzing hybridization. More particularly, the methods described herein allow for the determination of the presence of specific polynucleotides such as mutant genes in a sample, and may allow for a reliable identification and/or quantification of mutant genes in a sample.

More particularly, provided herein is a method for determining the presence of a mutant polynucleotide in a sample solution, said mutant polynucleotide differing from a target polynucleotide comprising a target sequence in one or more nucleotides of said target sequence, said method comprising contacting said sample solution as well as a reference solution comprising said target polynucleotide and essentially free of said mutant polynucleotide with a plurality of probes and comparing the hybridization intensities obtained for both samples and determining the presence of said mutant polynucleotide based thereon, wherein the different probes of said plurality of probes are characterized in that they are designed to by a varying complementarity to said target sequence. More particularly the varying complementarity to said target sequence is limited to a maximum of one or two non-complementary nucleotides with respect to the target sequence.

In particular embodiments the methods described herein comprise: (i) contacting said sample solution with a first plurality of probes, and obtaining first hybridization intensities for each of said first plurality of probes; (ii) contacting a reference solution comprising said target polynucleotide and essentially free of said mutant polynucleotide, with a second plurality of probes, and obtaining second hybridization intensities for each of said second plurality of probes;

and (iii) comparing said first hybridization intensities with said second hybridization intensities for corresponding probes for said sample and said reference solution and determining the presence of said mutant polynucleotide based thereon; wherein the method is characterized in that said first plurality of probes is identical to said second plurality of probes, and that the different probes of said plurality of probes are characterized by a varying complementarity to said target sequence.

In particular embodiments, step (iii) comprises analyzing the logarithm of said first hybridization intensities as a function of the logarithm of said second hybridization intensities for corresponding probes.

In further embodiments, the method comprises determining whether two or more parallel linear relationships can be distinguished between parts of said logarithm of said first hybridization intensities as a function of the logarithm of said second hybridization intensities.

In particular embodiments, said first and second plurality of probes each comprise: a perfect match probe for said target sequence and a variety of probes with one or two non-complementary nucleotides with respect to said target sequence, wherein each of said perfect match probe and each of said plurality of probes are provided on separate spots on a surface.

In certain embodiments of the methods provided herein, said reference solution is essentially free of any mutant of said target polynucleotide.

In particular embodiments, the method further comprises selecting probes of said second plurality of probes for which the hybridization has reached thermodynamic equilibrium.

In certain embodiments, the method further comprises determining the relative amount of said target polynucleotide and said mutated target polynucleotide in said sample solution. In particular embodiments, the method further comprises determining which of a plurality of candidate mutant polynucleotides is present in said sample solution.

In certain embodiments, said sample solution is prepared by: extracting DNA from a sample of interest; amplification of a target polynucleotide and mutants thereof contained in said DNA using a pair of primers of which one primer has a phosphate modification at its 5' end, thereby obtaining double stranded DNA; and digesting the 5' phosphate modified strands of said double stranded DNA using lambda exonuclease.

In particular embodiments of the methods provided herein, said hybridization intensities are induced by emission of a label associated with a hybrid formed by binding of said target polynucleotide or mutants thereof and said probes.

In certain embodiments, said label comprises a hybridization sequence complementary to a sequence on said mutant polynucleotide and said target polynucleotide outside said target sequence.

In particular embodiments of the methods provided herein, said first and second plurality of probes each comprise at least 100 probes.

In certain embodiments of the methods provided herein, said first plurality of probes and said second plurality of probes are provided on separate spots of a microarray.

Further provided herein are tools for determining the presence of a mutant of a target polynucleotide comprising a target sequence, in a sample solution. In particular embodiments, the tool is a kit. In particular embodiments, the tool is a kit for determining the presence of a mutant of a target polynucleotide comprising a target sequence in a sample solution, comprising a plurality of probes, wherein the plurality of probes comprises a perfect match probe for said target sequence and probes with one or two non-complementary nucleotides with respect to said target sequence. In further particular embodiments, the kit comprises a microarray having a plurality of microarray spots each of them comprising a probe, wherein the probes of said spots comprise a perfect match probe for said target sequence and a plurality of probes with one or two non-complementary nucleotides with respect to said target sequence.

In particular embodiments the kits provided herein also comprise a reference solution comprising said target polynucleotide, wherein said reference solution is essentially free of said mutant, preferably essentially free of any mutant of said target polynucleotide.

Further provided herein is a computer program product for performing, when executed on a computing device, a method for determining the presence of a mutant of a target polynucleotide in a sample solution as described herein.

The present methods and tools allow for a highly reliable detection, identification and quantification of mutations such as point mutations in a gene. The methods can provide a surprisingly low detection sensitivity. More particularly, the inventors have found that the method may allow for the detection of mutant genes in a mixture of mutated and non-mutated genes comprising less than 1% mutant. Due to the intrinsic parallel character of the microarray technology, the present method makes it possible to detect hundreds of different point mutations in a single run. The above and other characteristics, features and advantages of the concepts described herein will become apparent from the following detailed description, which illustrates, by way of example, the principles of the invention.

## BRIEF DESCRIPTION OF THE DRAWINGS

The following description of the figures of specific embodiments of the methods and instruments described herein is merely exemplary in nature and is not intended to limit the present teachings, their application or uses. Throughout the drawings, corresponding reference numerals indicate like or corresponding parts and features.

**Fig. 1** Illustrative plot of hybridization intensities obtainable using a mixture of wild type and a mutant K-RAS nucleotide versus the corresponding hybridization intensities obtained using a solution containing only the wild type. The data points form four branches, wherein the most deviating branch (mutation branch) contains information about the mismatching nucleotides associated to the mutant K-RAS nucleotide.

**Fig. 2** Schematic illustration of competitive hybridization in the case of a mixture between mutant and wild type targets hybridizing to probe sequences. The four different pictograms indicate the four different nucleotides and their shapes showing their complementarity as pairs. In this Figure, the pictograms are at the same position for all strands. The grouping of the probes, in term of branches, is dependent on the type of the nucleotide.

**Fig. 3** Plot of data obtained for a sample containing G13C mutant (5%) and wild type (95%) K-RAS nucleotides, showing a strongly deviating mutation branch.

**Fig. 4** Plot of data obtained for a sample containing G13D mutant (5%) and wild type (95%) K-RAS nucleotides, showing a strongly deviating mutation branch.

**Fig. 5** Concentration profile based on equation **5**, showing exp(p)-1 in function of the fraction of mutant G12A.

**Fig. 6** Concentration profile showing exp(p)-1 in function of mutant fraction for 12 different KRAS mutations. The dotted line represents a statistical power of 90%.

In the figures, the following numbering is used:

1 - reference branch; 2 - mutation branch; 3 - side branch.

## DETAILED DESCRIPTION OF THE INVENTION

While potentially serving as a guide for understanding, any reference signs used herein and in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to ensure one or more of the technical effects envisaged herein. It is to be understood that each value as used herein is itself also specifically, and preferably, disclosed. Typically, the term "about" should be read in this context.

The recitation of numerical ranges by endpoints includes all numbers and fractions subsumed within the respective ranges, as well as the recited endpoints.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

The term "polynucleotide" as used herein may include oligonucleotides and refers to polymer composed of nucleotide monomers, typically having a length of at least 10 nucleotides. Typically, the polynucleotides such as the target polynucleotides and probes referred to herein are single-stranded polynucleotides. As used herein, the term "polynucleotide" may include deoxyribonucleic acid (DNA), ribonucleic acid (RNA) or peptide nucleic acid (PNA).

The term "equilibrium" as used herein refers to thermodynamic equilibrium and indicates a situation wherein a steady state is obtained such that the number of conventional target-probe bindings does not substantially change over time. The term "non-equilibrium" or "non- equilibrium effects" refers to occurrence of a target-probe binding state that may change over time.

The term "free energy" as used herein refers the Gibbs free energy ($\Delta G$) or chemical potential. Where in embodiments analysis is performed as function of hybridization free energy, this includes analysis as function of $\Delta\Delta G$,

being the free energy difference between a perfect matching hybridization and a hybridization where the probe sequences have one or more internal mismatches.

The term "hybridization" as used refers to nucleic acid hybridization. This refers to the process of establishing a non-covalent sequence-specific interaction between two or more complementary strands of nucleic acids into a single hybrid. The strands of nucleic acids that may bind to their complement can for example be oligonucleotides, DNA, RNA or PNA. Nucleotides form the basic components of the strands of nucleic acids. Hybridization comprises binding of two perfectly complementary strands (in the Watson-Crick base-pairing senses), but also binding of non-perfect complementary strands. With a non-perfect complementary strand reference may be made to strands having a small number of non-complementary elements such as one, two or more non-complementary elements, preferably one or two non-complementary elements. In principle there is no limit to the number of non-complementary elements but the more non-complementary elements, the easier these are detectable.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are also envisaged herein, and form different embodiments, as would be understood by those in the art. For example, in the appended claims, any of the features of the claimed embodiments can be used in any combination. Provided herein are methods for analyzing hybridization. The methods allow for determining the presence of a mutant polynucleotide, also referred to herein as "mutant" in a sample solution. Accordingly, in a first aspect, the present application provides a method for determining the presence of a mutant polynucleotide in a sample solution. The term "mutant polynucleotide" or "mutant" as used herein refers to a polynucleotide having a sequence which differs from the sequence of a certain target polynucleotide in one or more nucleotides. It will be understood by the skilled person that the term "mutant" is not limited to sequences which are the result of a change in the target polynucleotide in a specific organism, tissue or cell but also include naturally occurring (i.e. evolutionary) sequence variants. More particularly in the context of the present application, these differences or mutations are located within a certain subsequence of the target polynucleotide, referred to herein as the "target sequence". Again, it will be understood that the "target sequence" is the sequence used as the reference sequence. In particular embodiments, the mutant polynucleotide only differs from the target polynucleotide in one or more nucleotides within the target sequence. Preferably, the mutant polynucleotide differs from the target polynucleotide in a limited number of nucleotides within the target sequence, preferably in at most two nucleotides, such as only in one nucleotide. Although the present method focuses on the interaction between a strand that initially is in a sample solution, and a strand that is bound to a surface, it is noted that hybridization may occur between nucleic acid strands that both are in solution. The strands initially present in the sample solution are typically referred to in the art as "target", whereas the strand which is to hybridize to the target is referred to as "probe". Accordingly, the mutant polynucleotide(s) and target polynucleotide referred to herein may both be considered as "targets". The probe may for example be a strand of oligonucleotides, DNA, RNA or PNA (partially) complementary to a target which may be present in the sample solution. Although the probe is preferably bound to a surface, the present methods may also be performed using probes in solution.

The methods described herein comprise measuring the degree of hybridization between a set of probes and polynucleotides of a sample solution and comparing this to the degree of hybridization of the same set of probes and a reference sample. Accordingly, the methods provided herein are based on the simultaneous detection of the degree of hybridization of a plurality of probes to a sample.

More particularly, the methods envisaged herein comprise

(i) contacting the sample solution with a first plurality of probes, and obtaining or detecting first hybridization intensities for each of said first plurality of probes; and
(ii) contacting a reference solution comprising said target polynucleotide and essentially free of mutants of said target polynucleotide, with an second plurality of probes; and obtaining or detecting second hybridization intensities for each of said second plurality of probes.

Although the methods envisaged herein may be used for the analysis of hybridization to probes in solution, it is preferred that the probes are provided on a surface.

Accordingly, in particular embodiments, the methods envisaged herein comprise

(i) contacting the sample solution with a first plurality of probes bound to a surface, and obtaining or detecting first hybridization intensities for each of said first plurality of probes; and

(ii) contacting a reference solution comprising said target polynucleotide and essentially free of mutants of said target polynucleotide, with an second plurality of probes bound to a surface; and obtaining or detecting second hybridization intensities for each of said second plurality of probes.

The order wherein contacting steps (i) and (ii) are performed is not critical. Accordingly, these steps can be performed in any order or even simultaneously.

The sample solution typically comprises a mixture of the target polynucleotide and a mutant polynucleotide, wherein the concentration of the mutant polynucleotide [$c(mut)$] is significantly smaller than the concentration of the target or wild type polynucleotide [$c(wt)$]. The ratio of these concentrations [$c(mut)/c(wt)$] is also referred to herein as the "relative concentration" of mutant polynucleotide in the sample solution. In preferred embodiments, the relative concentration of mutant polynucleotide in the sample solution is between 0.01 and 0.5, more preferably between 0.01 and 0.1. The relative concentration may also be expressed as a percentage, which refers to $100*[c(mut)/c(wt)]$.

The sample solution may be prepared using standard methods known in the art. This may include extracting DNA or other polynucleotides from a sample of interest, followed by amplification of certain fragments within the extracted DNA. Typically, amplification is performed using PCR (polymerase chain reaction). However, this results in double stranded DNA, whereas single-stranded DNA is preferred for the present methods. Indeed, hybridization of double-stranded DNA with nucleic acid probes is hampered by competition between the complementary non-target strand and the probe. Such competition can be avoided by degradation of the complementary strands, for example using lambda exonuclease. Lambda exonuclease is a processive enzyme that acts in the 5' to 3' direction, catalyzing the removal of 5' mononucleotides from duplex DNA. The preferred substrate is 5'-phosphorylated double stranded DNA. Accordingly, in certain embodiments, the preparation of the sample solution may comprise the steps of:

- extracting DNA from a sample of interest;
- amplification of a target polynucleotide and mutants thereof contained in said DNA using a pair of primers of which one primer has a phosphate modification at its 5' end; thereby obtaining double stranded DNA; and
- digesting the 5' phosphate modified strands of said double stranded DNA using lambda exonuclease.

Contacting steps (i) and (ii) are typically performed under conditions suitable for hybridization of the target polynucleotide to said probes. These conditions are typically also suitable for hybridization of the mutant to the probes, given the similarity between the target polynucleotide and mutant polynucleotide. The skilled person understands that relevant parameters for optimizing hybridization include hybridization time, temperature, and probe length. In preferred embodiments, the probes have a length ranging from about 20 to about 30 nucleotides.

In the methods described herein, the first and second plurality of probes form two identical probe sets, i.e. the probes of the first plurality are identical to the probes of the second plurality. Accordingly, for each probe of the first plurality of probes, there is a corresponding identical probe in the second plurality of probes. This allows for a direct comparison between the hybridization intensities for both sets. The terms "first plurality of probes" and "second plurality of probes" are also referred to herein as "first probe set" and "second probe set", respectively.

The probes of the first probe set (and therefore also the second probe set) are selected so that they provide a varying complementarity to the target sequence, more particularly so as to cover a range of hybridization intensities for the hybridization between the target polynucleotide and the probes. This may be obtained by providing different (single-stranded) probes having different binding affinities for the (target sequence of) the target polynucleotide. A hybridization probe may contain a hybridization sequence (intended for hybridization with the target and of which the sequence will be determined by the target) and a tail sequence, which may be used to hybridize to other sequences, for tagging of the probe, etc... Typically, the probe sets will include a plurality of mismatch (MM) probes having a hybridization sequence having one or more, preferably one or two, non-complementary nucleotides with respect to the target sequence. The hybridization sequence and the target sequence typically have the same length, i.e. contain the same number of nucleotides.

In certain embodiments of the present methods, the first and second probe set each comprise:

- a perfect match (PM) probe for said target sequence; and
- a variety of mismatch (MM) probes with respect to said target sequence;

wherein said perfect match probe and said each of said plurality of probes are preferably provided on separate spots on a surface. The term "perfect match probe" as used herein refers to a probe having a hybridization sequence which is completely complementary to the target sequence of the target polynucleotide. The term "mismatch probe" as used herein refers to a probe having a hybridization sequence which is non-complementary to the target sequence because the hybridization sequence comprises one or more non-complementary nucleotides with respect to the target sequence. In preferred embodiments, the MM probes comprise at most two non-complementary nucleotides. Thus, the MM probes preferably comprise either one or two non-complementary nucleotides.

The optimal number of probes required for the present methods may depend on various parameters such as the target sequence length and the amount and type of possible mutants expected in the sample. Typically, the first and second probe sets will each comprise at least 100 probes, preferably at least 500, at least 1000, or even more.

In the methods envisaged herein, the probes are preferably provided on a surface. Although the probes may be provided on any type of carrier, it is preferred that the probes are provided on a microarray. Thus, in particular embodiments of the methods described herein, the first and second plurality of probes are provided on separate spots of a microarray. A microarray as a hybridization platform contains a large number of probes which are immobilized on a solid surface. The probes are provided in spatially separated spots, wherein each spot comprises one (and only one) type of probe. Typically, each spot comprises only a few picomoles of each probe. Typical microarrays comprise hundreds or even thousands of spots. A plurality of microarray platforms suitable for use in the present methods are commercially available, and include but are not limited to the platform provided by Agilent, the GeneChips platform from Affymetrix or CodeLink Bioarray platform from Amersham Biosciences.

In preferred embodiments, the first and second plurality of probes may be provided on the same microarray. This can facilitate comparing the hybridization intensities for the two sets of probes.

The methods as envisaged herein involve the comparison of the hybridization of a set of probes with a sample and with a reference solution. The reference solution is characterized in that it comprises the target polynucleotide. Moreover, the reference solution is typically prepared such that any hybridization intensity (above the background signal) detected upon contacting the reference solution to the probes is attributable to the hybridization of the target polynucleotide to the probes. This can be achieved in various ways.

In preferred embodiments, the reference solution is free from the mutant polynucleotide of interest. In further embodiments, the reference solution is essentially free of any strands comprising one or more mutations in the target sequence. In this way, it can be ensured that essentially all of the hybridization intensity results from hybridization of the target polynucleotide to the probes. The reference solution may still contain polynucleotide strands which do not comprise the target sequence, provided that they do not significantly hybridize with the plurality of probes. Such polynucleotide strands may include "barcode" strands which can be used for labeling the target polynucleotide (see further). In certain embodiments however, essentially all of the strands of the reference solution actually comprise the target sequence or even completely correspond to the target nucleotide. In certain embodiments, at least 99.9%, more preferably at least 99.95% of all strands present in the reference solution comprise the target sequence or even correspond to the target polynucleotide.

Additionally or alternatively, the target polynucleotide present in the reference solution may be labeled with a certain marker (see further), wherein the target polynucleotide is the only polynucleotide in the reference solution which is labeled with said marker.

The hybridization intensity is a value representing the fraction of a certain probe which is hybridized. In the methods described herein, detection of hybridization intensity may be performed using a marker associated with the formed hybrid, such as for example a fluorescence marker or a radio-active marker, or other markers known in the art. In preferred embodiments, the marker used for the sample solution is the same as the marker used for the reference solution. However, this is not critical for the present methods. Accordingly, different markers may be used for the sample solution and the reference solution. In certain embodiments, the detection of the hybridization intensity may be performed using a label-free method, such as surface-enhanced Raman spectroscopy.

Typically in hybridization experiments, intensity of the radiation or fluorescence provided by the markers is detected and representative for the number of hybrids formed. Thus, in certain embodiments, the hybridization intensities may be induced by emission of a label associated with a hybrid formed by binding of the target polynucleotide or mutant thereof and said probes. Suitable fluorescence markers for the present methods include, but are not limited to, Cy3 and Cy5, which are dyes of the cyanine dye family.

The markers or labels may be associated to the target or mutant polynucleotide prior to or after hybridization. In some embodiments, a fluorescent dye or other marker compound may be associated directly to the target or mutant thereof. In other embodiments, the marker compounds may be associated to the target or mutant thereof in an indirect manner, for example via a "barcode", which is a strand having a hybridization sequence which is complementary to a tail sequence which is present on the mutant polynucleotide of interest and on the target polynucleotide, thereby allowing hybridization between the barcode and target (or mutant thereof), and therefore indirect coupling of the fluorescence marker or other marker to the target. More particularly, the strand hybridizes to a tail sequence outside the target sequence of the target polynucleotide, such that it does not significantly interfere with the hybridization between the targets and the probes.

In an analysis step (iii) of the present methods, the first hybridization intensities are compared with the second hybridization intensities for corresponding probes. Thus, the intensity of each probe of the first probe set may be compared to the intensity of the corresponding probe of the second probe set. Based on the comparison of the hybridization intensities, the presence of one or more mutant polynucleotides can be determined.

Indeed, the present inventors have found that by comparing the hybridization intensities of hybridization experiments on a sample solution as well as a reference solution using two identical probe sets as described herein, it is possible to identify mutant polynucleotides in a mixture of mutant and wild type polynucleotide at surprisingly low concentrations of the mutant relative to the wild type.

Specific ways to detect and/or identify mutant polynucleotides in a sample solution will be explained using theoretical concepts based on the thermodynamics of DNA hybridization. The skilled person will understand that this also applies, mutatis mutandis, to nucleotides other than DNA.

In a microarray data analysis, each probe intensity (hybridization intensity) is associated to a signal from a spot. A spot is a local space on the microarray slide that contains a large number of identical sequences corresponding to a certain type of probe in the probe set. Therefore, each spot represents a single type of probe. Each of these identical sequences within a spot is supposed to be hybridized to a floating target sequence depending on the affinity between the two sequences. This affinity is sequence dependent and determines the fraction of hybridized probes in a spot.

In a hybridization model with Langmuir isotherm (Hooyberghs et al., Nucleic Acids Res. 2009, 37, e53), the relationship between the detected intensity and the hybridization affinity for the target-probe hybrid can approximately be written as equation (1) (assuming the hybridization between the target and probe sequences are in thermodynamic equilibrium, and that the fraction of hybridized probes in a microarray spot is such that the detected intensities are significantly above the background yet far from saturation):

$$I = A.c.e^{-\Delta G/RT} \qquad (1)$$

wherein $I$ is the detected hybridization intensity, $A$ is a proportionality factor for the intensity, c target concentration in solution, $\Delta G$ hybridization free energy as a sequence dependent measure for the affinity, R the ideal gas constant, and T experimental temperature.

This equation can be calculated for each spot of the microarray, i.e. for each probe type in a probe set.

The free energy $\Delta G$ can be rescaled to the value of the free energy of the perfect match (PM) hybrid $\Delta G_{PM}$. The PM hybrid refers to the hybrid formed by the target sequence or target polynucleotide and the PM probe. This rescaled free energy is denoted as $\Delta\Delta G \equiv \Delta G - \Delta G_{PM}$. Therefore, the rescaled free energy for the PM hybrid $\Delta\Delta G_{PM}$ is zero. The probe set can be designed such that each available probe (except the PM probe) contains a mismatch (DNA defect) against the wild type. Follow the Nearest-Neighbor model (Bloomfield VA et al., "Nucleic Acids Structures, Properties and Functions, University Science Books, Mill Valley, 2000) for the free energy of DNA, $\Delta\Delta G$ can be interpreted as the measure for affinity penalty due to mismatch.

In the case of a hybridization where the sample contains mixture of target sequences i.e. a wild type sequence and a mutant such as in a clinical situation, there will be a competition between the two target sequences to hybridize to a single probe. To describe this competitive hybridization, equation 1 can be extended to:

$$I = I(wt) + I(mut) = A.c(wt).e^{-\Delta Gwt/RT} + A.c(mut).e^{-\Delta Gmut/RT} \qquad (2)$$

Wherein $I(wt)$ is the wild type contribution to the total signal and $I(mut)$ is the mutant contribution, $c(wt)$ is concentration of wild type target sequence, $c(mut)$ is concentration of mutant target sequence, $\Delta G(wt)$ is free energy of the wild type and $\Delta G(mut)$ is free energy of the mutant.

In the methods described herein, intensity data from a mixture experiment [$I(mix)$] may be compared with reference intensity data [$I(ref)$] from a microarray experiment that only contains wild type target sequence.

More particularly, step (iii) of the present methods comprises analyzing the logarithm of the first hybridization intensities as a function of the logarithm of the second hybridization intensities for corresponding probes. If a mutation of the target polynucleotide is present in the sample solution, a plot of the logarithm of the of the first hybridization intensities in function of the logarithm of the second hybridization intensities for corresponding probes will show several branches which contain information about the mutant present in the sample (see further). It will be evident to the skilled person that similar results can be obtained by using the actual hybridization intensities for the plot while using logarithmic scales for the axes.

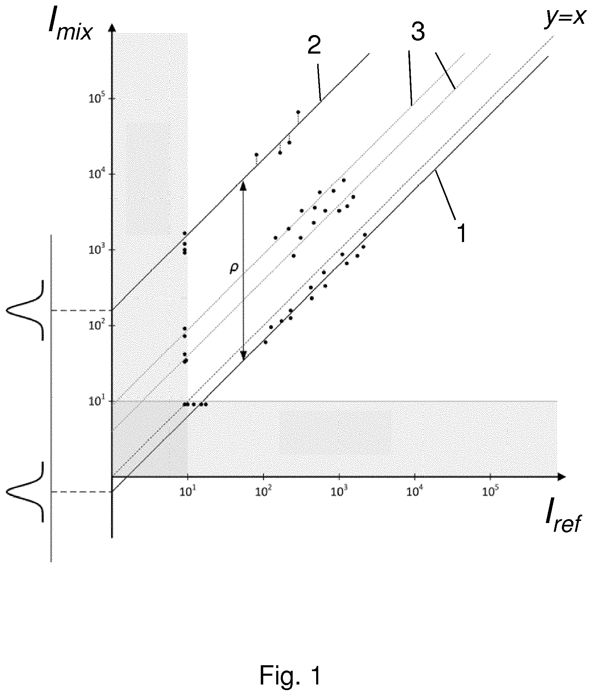

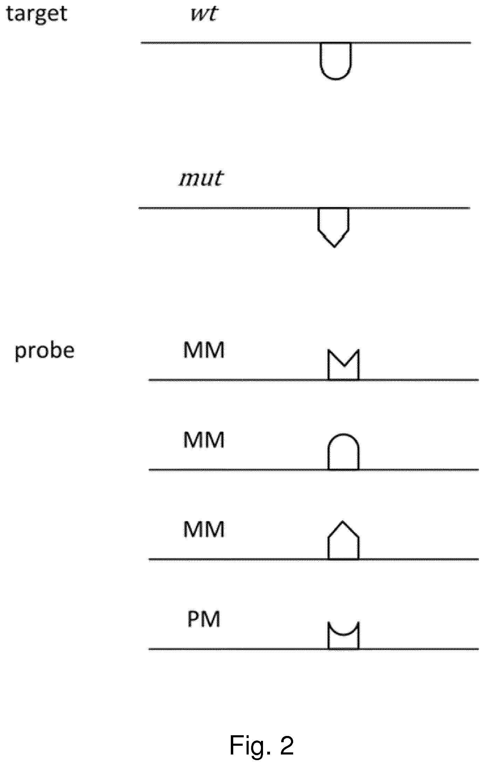

Fig. 1 shows an illustrative plot of $I(mix)$ against $I(ref)$ for a simple theoretical microarray experiment, using axes with a logarithmic scale. After filtering out data points within the background noise region, the data splits into four groups or "branches": one reference branch (1), a mutation branch (2), and two side branches (3). These branches can be explained through Fig. 2, which illustrates target and probe sequences, with pictograms depicting nucleotide at the same position on each sequence. The shape of the pictograms indicates the Watson-Crick complementarity as pairs. The mutant target has one different nucleotide compared to the wild type. Four probes are provided with all possible nucleotides. It is clear that if the nucleotides are complementary to each other, they will have much higher affinity to bind. Therefore, the PM probe will have higher affinity to bind with the wild type such that for this probe $I(wt)>>I(mut)$. These probes will be part of the reference branch. On the other hand, one of the mismatch (MM) probed is complementary to the mutant and therefore will have higher affinity to bind to the mutant target, such that for this probe $I(wt)<<I(mut)$. These probes will belong to the mutation branch. The nucleotides of the other two probes contain a mismatch to both the wild type and the mutant target, so they will be lying in between the reference and the mutation branch.

In order to identify the type of mutation, the probes belonging to the mutation branch can be analyzed. The difference between the two intensity datasets (one dataset corresponding to the reference sample containing wild type only, and one to the sample containing the mixture) can be denoted as p, wherein:

$$\mathrm{Ln}[I(mix)/I(ref)] \equiv \rho \qquad (3)$$

p can be measured by determining the distance between two lines drawn parallel to the unit line y=x that minimize the sum of the vertical distances between the data points of each branch to their respective line, as shown in Fig. 1.

Accordingly, in certain embodiments, step (iii) of the present methods may comprise determining whether two or more parallel linear relationships can be distinguished between (parts of the) logarithm of the first hybridization intensities as a function of the logarithm of the second hybridization intensities. In further embodiments, the methods may comprise determining the distance p between these linear relationships.

This distance p between the reference and mutation branch can be expressed mathematically through the following equation:

$$\rho = \ln[1 + (c_{mut}/c_{wt}).e^{-\Delta\Delta G/RT}] \qquad (4)$$

wherein $\Delta\Delta G = \Delta G(mut) - \Delta G(wt)$. Using Equation 4 the distance p can be related to the relative concentration of the mutant [$c(mut)/c(wt)$] and the free energy difference between mutant and wild type sequences $\Delta\Delta G$. In this context, $\Delta\Delta G$ is again the measure of affinity penalty due to mismatches. However, this time, the mismatching nucleotides are between the mutant and wild type. Therefore, Equation 4 shows that p can be used to measure affinity.

Furthermore, it is clear from equation 4 that the present method may be used for determining the relative amount of the mutant and the target polynucleotide [$c(mut)/c(wt)$] in the sample solution.

The methods described herein may also be used for testing whether a specific mutation is present in a sample. For such tests, the hybridization intensities may be analyzed using statistical methods. As discussed above, information of a mutant in a sample is available on a mutation branch on a hybridization intensity plot as shown in Fig. 1. The existence of a mutation branch can be tested using a two-sample t-test, wherein the two groups are the data of the reference and the mutation branch. The distance p between the two groups can be estimated by projecting the data points of the groups to the y-axis parallel to the unit line y=x, as shown in Fig. 1. This is a two-sample one-sided t-test, with unequal sample sizes and unequal standard deviations. The null hypothesis [H(0)] is p =0. The alternative hypothesis [H(a)] is p >0. However, solely testing this hypothesis may not be reliable as possible mutations on the same location can also lead to significant p-values. To determine which mutation is actually present in a sample, the different distances p of all possible mutations can be determined, wherein the mutation for which p is the highest is considered to be the one corresponding to Equation 4.

Thus, in certain embodiments, the present methods may comprise determining which of a plurality of candidate mutant polynucleotides is present in the sample solution.

In certain embodiments, the hybridization intensities certain probes or spots may be excluded from the comparison in step (iii). For example, probes or spots may be excluded from further analysis because the corresponding hybridization intensity is either too low (not significantly above the background signal) or too high (above the saturation level). Fig. 1 shows the areas in the plot corresponding to the background noise region. Data points within these areas are preferably excluded from the analysis.

As a further example, certain probes or spots may be excluded from further analysis because the hybridization for these spots has not reached equilibrium. However, it is preferred that the hybridization experiments are performed under such conditions that hybridization has reached equilibrium, e.g. by selecting suitable probe lengths, temperatures, and hybridization time. A method for determining for which probes or spots hybridization has reached equilibrium is described in international patent application WO 2011/035801.

In particular embodiments of the present methods, the sample solution may further be contacted to a third and even further pluralities of probes, which are identical to the first and second plurality of probes. In this way, the hybridization intensities of the corresponding probes may be averaged, which may further improve the reliability of the present methods. Similarly, also the reference sample may be contacted with further pluralities of probes, wherein the intensities of corresponding probes are averaged. Accordingly, the skilled person will understand that the step (iii) of comparing the hybridization intensities may comprise averaging the results of various probe sets. Further provided herein are tools for performing the methods described herein. In particular embodiments, the tools are kits, i.e. combinations of reagents. More particularly, provided herein is a kit for determining the presence of a mutant of a target polynucleotide comprising a target sequence in a sample, said kit comprising a plurality of probes comprising a perfect match probe for said target sequence and a plurality of probes with one or two non-complementary nucleotides with respect to said target sequence. In particular embodiments the kits comprise more than one set of said plurality of probes. In further particular embodiments, the tools provided herein comprise a microarray comprising at least two identical probe sets, each comprising a perfect match probe for said target sequence and a plurality of probes with one or two non-complementary nucleotides with respect to said target sequence. In yet further embodiments, the tools further comprise a reference solution comprising said target polynucleotide, wherein said reference solution is essentially free of mutants of said target polynucleotide differing from a target polynucleotide comprising a target sequence in one or more nucleotides of said target sequence. Thus in particular embodiments, the kits comprise:

- a reference solution comprising said target polynucleotide, wherein said reference solution is essentially free of mutants of said target polynucleotide; and
- a microarray comprising at least two identical probe sets, each comprising a perfect match probe for said target sequence and a plurality of probes with one or two non-complementary nucleotides with respect to said target sequence.

The features of the reference solution, target polynucleotide, target sequence, microarray, and probe sets as described above for the methods, are also applicable to the kit.

Further provided herein is a computer program product for performing, when executed on a computing device, at least a part of a method for determining the presence of a mutant of a target polynucleotide as described herein. For example, the computer programs may be configured for receiving and analyzing hybridization intensities according to the methods described herein. For example, the computer program may be configured to compare the intensity of corresponding probes of the first and second probe set, and to identify reference and mutation branches as described herein. In particular embodiments, the computer program product being configured for receiving first hybridization intensities for a sample solution, receiving second hybridization intensities for a reference solution comprising said target polynucleotide and analyzing the logarithm of said first hybridization intensities as a function of the logarithm of said second hybridization intensities for corresponding probes and determining the presence of a mutant polynucleotide in said sample solution based thereon.

The software may further be configured to perform a statistical analysis of the hybridization intensity data in order to determine which of a plurality of candidate mutants is present in a sample solution. In certain embodiments, the computer programs may further be configured for designing suitable probe sets based on information of the target sequence and/or mutations thereof.

In case of implementation or partly implementation as software, such software may be adapted to run on suitable computer or computer platform, based on one or more processors. The software may be adapted for use with any suitable operating system. The computing means may comprise a processing means or processor for processing data.

A further tool provided herein is a device configured for carrying out the methods provided herein. More particularly the device comprises the combination of the necessary hardware and software for carrying out the different steps of these methods. The device may comprise hardware, in the form of reaction vessels and feeds for reagents connected thereto and a detection unit, which can ensure the contacting a sample solution with a first plurality of probes, hybridization of the sample solution with the first plurality of probes and measurement of first hybridization intensities for each of said first plurality of probes. The device may further comprise a parallel set of reaction vessels, feeds for reagents connected thereto and detection unit which allow the contacting of a reference solution with a second plurality of probes, hybridization between the reference solution and the second plurality of probes and measurement of the second hybridization intensities for each of said second plurality of probes; alternatively, the device may be configured to perform the steps on the reference solution subsequently to the first set of steps for the sample solution using some or all of the same hardware. Moreover, the device comprises a processing unit provided with the necessary software for performing the analysis step involving the comparison of the first and second measurements and optionally a display unit to present the results of said analysis to a user. In particular embodiments, the results are displayed as information on the presence of a mutant polynucleotide in the sample solution.

**EXAMPLES**

The following examples are provided for the purpose of illustrating the claimed methods and applications and by no means are meant and in no way should be interpreted to limit the scope of the present invention.

The inventors have applied the present method for the detection, identification and quantification of hotspot point mutations in the K-RAS oncogene, which is an important genetic marker for colorectal and lung cancer diagnostics and treatment stratification.

**1. Materials and methods**

*1.1 Sample preparations and experimental setup*

Several sets of hybridization experiments were performed in this study.

In a **first set** of experiments (gBlocks experiments), mixtures of wild-type KRAS ssDNA and mutant KRAS ssDNA were used. To obtain ssDNA mixtures, a PCR reaction was performed on double-stranded sequence-verified gBlocks® Gene Fragments (obtained from Integrated DNA Technologies, Leuven, Belgium), further referred to herein as "gBlocks". gBlocks sequences of the 12 most commonly reported KRAS mutations were used: G12C, G12S, G12R, G12D, G12A, G12V, G13C, G13S, G13R, G13D, G13A, and G13V. Mixtures were made with gBlocks wild-type DNA and contained 5% of mutant DNA.

The PCR reaction mixture comprised $0.4\mu$M forward (5'-GTCCTGCACCAGTAATATGC-3' SEQ ID NO:1) and $0.4\mu$M reverse (5' - CTGGCGTCATAGCTGTTTCCTGTGTGAGTATTAACCTTAT GTGTGACA-3' (SEQ ID NO:2)) primers (Eurogentec, Seraing, Belgium), 2mM MgSO$_4$, 0.2mM of each deoxyribonucleoside triphosphate (dNTP), 2 U Platinum Taq DNA High-Fidelity Polymerase (Life Technologies, Ghent, Belgium), and 0.5ng gBlocks DNA in a final volume of $50\mu$l. The reverse primer has a phosphate modification at the 5' end.

The DNA was amplified through 35 cycles (95°C, 30s; 55°C, 30s; 72°C, 30s) with a Verti® thermal cycler (Life technologies). Amplicons were purified using Qiagen PCR purification kit (Qiagen, Hilden, Germany), according to manufacturer's protocol. A Lambda exonuclease treatment (Fermentas, St.Leon-Rot, Germany) was performed on the purified PCR product according to manufacturer's protocol. The obtained ssDNA was analyzed on a FlashGel DNA system (Lonza, Slough, UK), and concentration was measured in a NanoDrop spectrophotometer. 10nM ssDNA was used in the microarray experiments. Dilutions for the **second set** of experiments to study the detection limit were made by mixing the available gBlocks ssDNA mutant G12A, and wild-type ssDNA, except for one experiment that uses a pure mutant sample, to the total of 5nM concentration. Exemplary concentrations of mutant DNA for the samples are 0.1024%, 0.256%, 0.64%, 4%, 10%, and 100% (relative mutant concentration).

Finally we tested **real clinical samples.** For this set of experiments, we obtained blind coded formalin-fixed paraffin embedded (FFPE) colon carcinoma samples from the Center of Medical Genetics Ghent. The DNA was extracted using a Gentra Puregene Tissue Kit (Qiagen) according to manufacturer's protocol. 100ng DNA was used in the PCR reaction. Samples were also sequenced by Sanger sequencing for KRAS status. The total concentration of each sample is 10nM.

*1.2 Microarray experiments*

The microarray experiments were performed using a commercially available Agilent platform while followed a standard protocol with Agilent products. Each hybridization mixture contains a Cy3-labeled Barcode (Cy3-5'-AAAAACTGGCGTCATAGCTGTTTCCTGTGTGA-3' (SEQ ID NO:3)) diluted in nuclease-free water to a final concentration of $0.05\mu$M together with ssDNA (various concentrations), $5\mu$l 10x blocking agent and $25\mu$l 2x GEx hybridization buffer HI-RPM. The hybridization mixture was centrifuged at 13000 rpm for 1 minute and each microarray of the $8\times15$K custom Agilent slides was loaded with $40\mu$l of the mixture. The hybridization occurred in an Agilent oven at 65°C for 17h with rotor setting 10 and the washing was performed according to the instructions of the manufacturer. The arrays were scanned on an Agilent scanner (G2565BA) at $5\mu$m resolution, high and low laser intensity and further processed using Agilent Feature Extraction Software (GE1 v5 95 Feb07) that performs automatic gridding, intensity measurement, background subtraction and quality checks.

*1.3 Probe set design*

A custom designed probe set was constructed for the microarray experiments to study the region around codon 12 and 13 of exon 2 of the K-RAS gene.

Based on previous experiments (Hooyberghs J et al., Phys. Rev. E, 2010, 81, 012901) it was estimated that a suitable probe-target affinity would be achieved for probes of a length of 23 nucleotides. This results in the wild type target sequence of interest shown in Table 1. From this sequence, a probe set was designed (see Table 1). This probe set contains one perfectly matching (PM) probe against the target wild type. The rest of the probes contain all possible single or double mismatches (1MMor 2MM) against the wild type target, avoiding the free energy penalty coming from interaction between two mismatches and a mismatch located close to the edge of the helix structure (Hadiwikarta WW et al., Nucleic Acids Res. 2012, 40, e138). Each probe was replicated eight times and the median over these replicates was used for data analysis.

*1.4 Defining mutation detection limit*

To define the detection limit of the present method, the minimum p was estimated for which a mutation can still be detected. This was done via power calculation for a one-sided two sample t-test with unequal sample size and unequal standard deviation. This calculation was programmed using simulation. The resulting curves were smoothed using lowess method.

**Table** 1 - Examples of probes used in the experiments. The probe set contains one type of probe that is perfectly matching to the target, whereas the other probes contain one or two mismatches against the target. Mismatches close to each other or near the boundaries of the sequence are avoided.

| TARGET | | SEQ ID |
|---|---|---|
| Wild type | 5'-GTTGGAGCTGGTGGCGTAGGCAA-3' | 4 |
| **PROBE** | | |
| PM | 3'-CAACCTCGACCACCGCATCCGTT-5' | 5 |
| 1 MM | 3'-CAAC**G**TCGACCACCGCATCCGTT-5' | 6 |
| | 3'-CAAC**A**TCGACCACCGCATCCGTT-5' | 7 |
| | 3'-CAAC**T**TCGACCACCGCATCCGTT-5' | 8 |
| | 3'-CAACC**C**CGACCACCGCATCCGTT-5' | 9 |
| | 3'-CAACC**G**CGACCACCGCATCCGTT-5' | 10 |
| | 3'-CAACC**A**CGACCACCGCATCCGTT-5' | 11 |
| | ... | |
| 2 MM | 3'-CAAC**G**TCGA**G**CACCGCATCCGTT-5' | 12 |
| | 3'-CAAC**G**TCGA**A**CACCGCATCCGTT-5' | 13 |
| | 3'-CAAC**G**TCGA**T**CACCGCATCCGTT-5' | 14 |
| | 3'-CAAC**G**TCGAC**G**ACCGCATCCGTT-5' | 15 |
| | 3'-CAAC**G**TCGAC**A**ACCGCATCCGTT-5' | 16 |
| | 3'-CAAC**G**TCGAC**T**ACCGCATCCGTT-5' | 17 |
| | ... | |

**2. Experimental results**

*2.1 gBlocks experiments*

In the gBlocks experiments, all 12 mutations were tested for each sample. The maximum -log10(p-value) and the maximum $\rho$ (enclosed in brackets) at each codon position for all available samples are displayed in Table 2. The last column displays the mutation that is linked to this maximum $\rho$.

**Table 2** - Summary results for the gBlocks experiments, showing the maximum -log10(p-value) and the maximum ρ (enclosed in brackets) for each codon position where a mutation might occur. The last column displays the mutation that is linked to this maximum ρ.

| Sample | pos-9 | pos-10 | pos-12 | pos-13 | Mutation found |
|---|---|---|---|---|---|
| 5% Mut G12C | 9.29 (2.64) | 0.78 (0.03) | 0.28 (0.03) | 0.51 (0.05) | G12C |
| 5% Mut G12S | 13.92 (2.71) | 0.21 (0.35) | 0.03 (0.33) | 0.56 (0.76) | G12S |
| 5% Mut G12R | 10.19 (4.24) | 1.37 (0.09) | 1.31 (0.10) | 1.35 (0.21) | G12R |
| 5% Mut G12D | 1.24 (0.05) | 20.00 (1.63) | 0.57 (0.03) | 0.28 (0.05) | G12D |
| 5% Mut G12A | 1.53 (0.14) | 8.96 (3.40) | 1.74 (0.08) | 0.87 (0.04) | G12A |
| 5% Mut G12V | 0.38 (0.14) | 12.17 (3.92) | 0.14 (0.11) | 1.17 (0.20) | G12V |
| 5% Mut G13C | 0.72 (0.06) | 1.20 (0.09) | 10.49 (3.20) | 0.98 (0.14) | G13C |
| 5% Mut G13S | 0.12 (0.10) | 0.23 (0.05) | 16.51 (2.42) | 0.31 (0.13) | G13S |
| 5% Mut G13R | 0.40 (0.02) | 1.86 (0.08) | 13.70 (4.42) | 0.25 (0.27) | G13R |
| 5% Mut G13D | 0.13 (0.37) | 0.09 (0.51) | 0.16 (0.37) | 9.51 (3.05) | G13D |
| 5% Mut G13A | 0.09 (0.14) | 1.88 (0.11) | 0.62 (0.03) | 12.57 (3.97) | G13A |
| 5% Mut G13V | 0.52 (0.22) | 0.28 (0.07) | 0.77 (0.06) | 13.30 (3.77) | G13V |

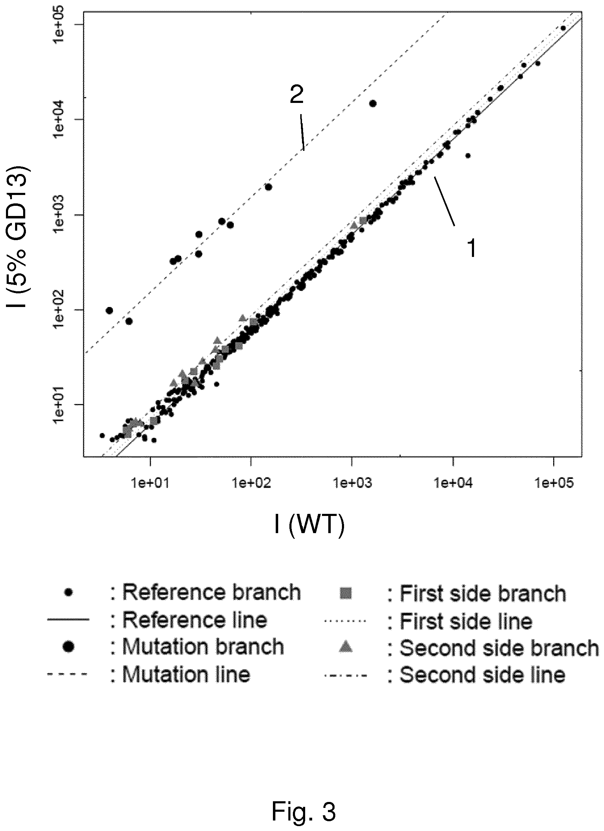

As an example, Fig. 3 presents the graphical result when testing mutation G13C. The mutation branch clearly has higher intensities on the y-axis. As the measure for statistical significance, instead of using the p-values as a result from the t-test, -log10(p-value) values are used in order to avoid small numbers. Using a p-value of 0.05 as the significance measure, log10(0.05)≈1.3. The resulting -log10(p-value) when testing mutations G13S, G13R and G13C are 2.02, 4.84, and 10.49 (i.e. all three are significant); and the corresponding p values are 0.14, 0.33, and 3.20, respectively.

However, the two lowest -log10(p-value) have low corresponding ρ values and are representing the side branches. The highest -log10(p-value) and also the highest p are associated to G13C, which indeed is the mutation present in the sample.

The tests of all other 9 mutations end up with non-significant -log10(p-value) and very small ρ values.

These results indicate that the present method not only allows for detecting the presence of mutations in a sample, but also allows for identifying which of a number of alternative mutations is present in the sample.

*2.2 Experiments with clinical samples*

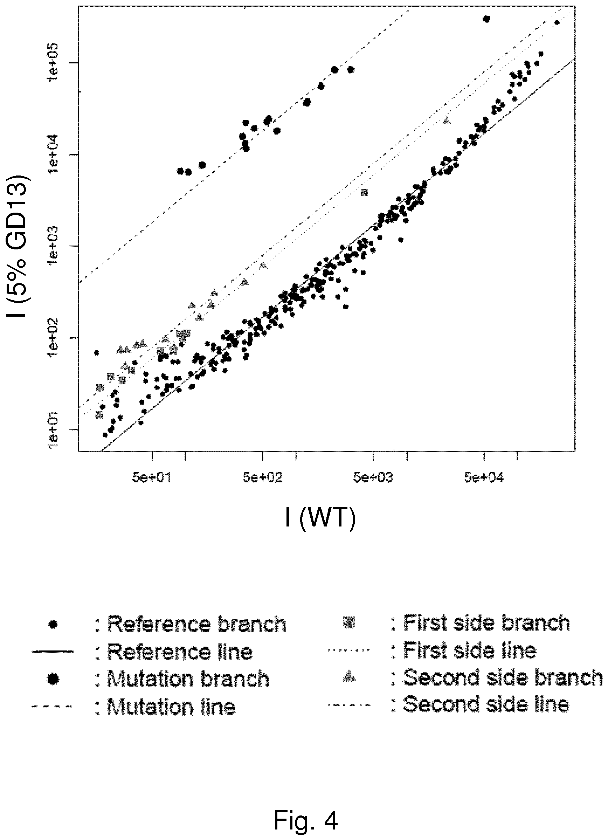

Blinded tests were performed on real clinical samples taken from patients, more particularly formalin-fixed, paraffin-embedded (FFPE) clinical samples. Convincing results were obtained for each sample using the present method. Fig. 4 shows the results for one of the samples as an example. In this case, the results clearly indicated the presence of mutant G13D with -log10(p-value) = 16.71 and p= 4.679. Results from other experiments also displayed strong mutation branches which allowed for identifying the mutations present in the samples. All results of the blinded tests correspond to results measured via reference tests. These results show that the present method allows for identifying mutations in real clinical samples.

*2.3 Linearity of concentration profile*

In order to get an estimate of the detection limit of the method, microarray experiments were performed on a number of samples containing a known mutant but with incrementally increased mutant concentration. In this study, six different concentrations of the same mixture from the mutant G12A were tested.

Equation **4** discussed above can be rearranged to equation **5**:

$$e^{\rho} - 1 = (c_{mut}/c_{wt}) . e^{-\Delta\Delta G/RT} \qquad (5)$$

From equation **5**, it is clear that there is a linear relation (in log scale) between p and the fraction mutant [*c(mut)/c(wt)*], wherein the slope of this linear relation is exp(-ΔΔG/RT). With RT constant, the value that determines the slope is ΔΔG.

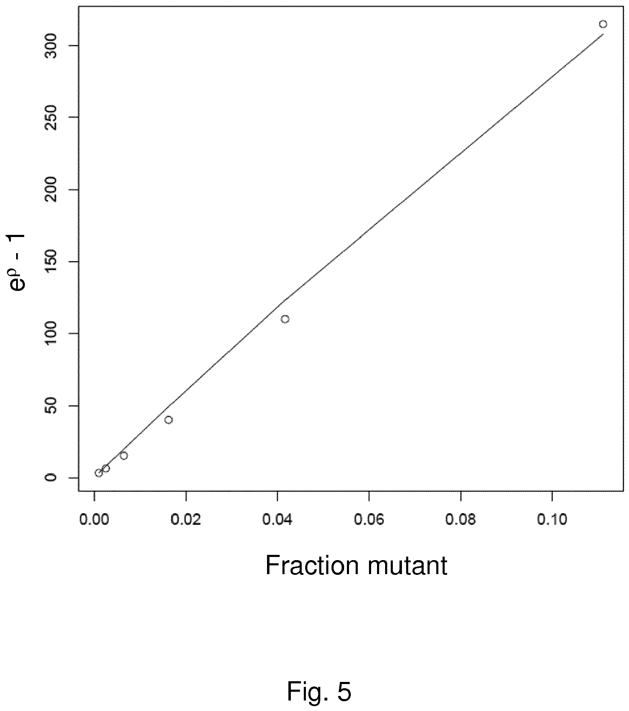

A concentration profile can be made (Fig. 5) wherein [e(exp)(ρ) - 1] is plotted versus the fraction mutant fraction. Fig. 5 clearly shows that the obtained data perfectly fits the theoretical linear relationship according to equation **5**.

From equation **5** it is further clear that the linear relationship is going through the origin (0,0), which means that only one sample is needed for each mutation in order to estimate the ΔΔG and thus the whole concentration profile for each mutation.

*2.4 Defining the detection limit*

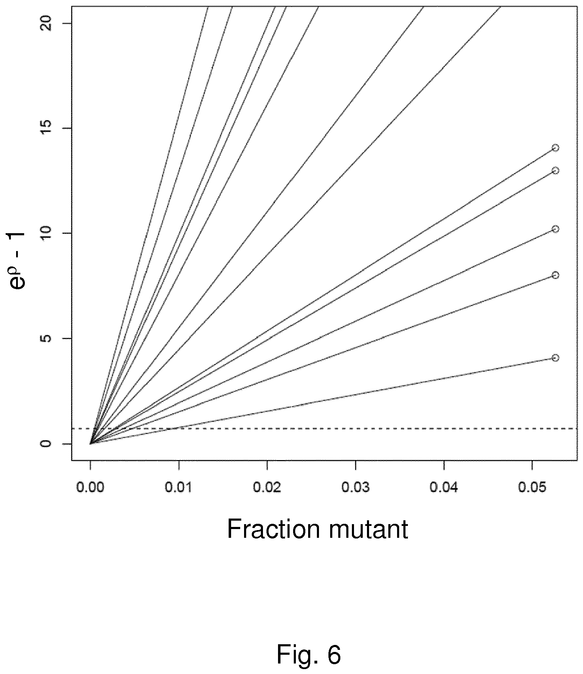

After p was determined for each mutation, the concentration curve for each mutation was plotted as shown in Fig. 6. From the slope of the concentration curves, ΔΔG can be derived for each mutation. Mutations having a weaker ΔΔG have a smaller slope, and are more difficult to detect in small concentrations using a t-test as described above.

The minimum p for which the present method still allows for the detection of the mutation (or reject the null hypothesis of no mutation) with enough statistical power was determined based on an estimate of the sample size and the standard deviation for the reference branch and the mutation branch, setting the statistical power conservatively to 90%. The minimum p is drawn in Fig. 6 as a horizontal dotted line, which shows that for any one of the mutations, the present method allows for detecting a relative mutant concentration as low as 1%.

In conclusion, using a dilution series of mutant and wild type mixture samples the inventors have shown that the method described herein can provide a low detection sensitivity (below 1% of mutant sequence). The inventors have further demonstrated that the method can be applied to real paraffin embedded clinical samples, showing it can deal with the limited quality and heterogeneity of the tissue material. Due to the intrinsic parallel character of the microarray technology, this approach makes it possible to detect hundreds of different point mutations in a single run.

**Claims**

**1.** A method for determining the presence of a mutant polynucleotide in a sample solution, said mutant polynucleotide differing from a target polynucleotide comprising a target sequence in one or more nucleotides of said target sequence, said method comprising:

(i) contacting said sample solution with a first plurality of probes, and obtaining first hybridization intensities for each of said first plurality of probes;
(ii) contacting a reference solution comprising said target polynucleotide and essentially free of said mutant polynucleotide, with a second plurality of probes, and obtaining second hybridization intensities for each of said second plurality of probes;

and

(iii) analyzing the logarithm of said first hybridization intensities as a function of the logarithm of said second hybridization intensities for corresponding probes and determining the presence of said mutant polynucleotide based thereon;

wherein said first plurality of probes is identical to said second plurality of probes, and wherein the different probes of said plurality of probes are **characterized by** a varying complementarity to said target sequence.

**2.** The method according to claim 1, wherein said first and second plurality of probes each comprise:

- a perfect match probe for said target sequence; and
- a variety of probes with one or two non-complementary nucleotides with respect to said target sequence;

wherein each of said perfect match probe and each of said plurality of probes are provided on separate spots on a surface.

**3.** The method according to claim 1 or 2, wherein said reference solution is essentially free of any mutant of said target polynucleotide.

**4.** The method according to any one of claims 1 to 3, comprising determining whether two or more parallel linear relationships can be distinguished between parts of said logarithm of said first hybridization intensities as a function of the logarithm of said second hybridization intensities.

5. The method according to any one of claims 1 to 4, further comprising selecting probes of said second plurality of probes for which the hybridization has reached thermodynamic equilibrium.

6. The method according to any one of claims 1 to 5, further comprising determining the relative amount of said target polynucleotide and said mutated target polynucleotide in said sample solution.

7. The method according to any one of claims 1 to 5, further comprising determining which of a plurality of candidate mutant polynucleotides is present in said sample solution.

8. The method according to any one of claims 1 to 7, wherein said sample solution is prepared by:

- extracting DNA from a sample of interest;
- amplification of a target polynucleotide and mutants thereof contained in said DNA using a pair of primers of which one primer has a phosphate modification at its 5' end, thereby obtaining double stranded DNA; and
- digesting the 5' phosphate modified strands of said double stranded DNA using lambda exonuclease.

9. The method according to any one of claims 1 to 8, wherein said hybridization intensities are induced by emission of a label associated with a hybrid formed by binding of said target polynucleotide or mutants thereof and said probes.

10. The method according to claim 9, wherein said label comprises a hybridization sequence complementary to a sequence on said mutant polynucleotide and said target polynucleotide outside said target sequence.

11. The method according to any one of claims 1 to 10, wherein said first and second plurality of probes each comprise at least 100 probes.

12. The method according to any one of claims 1 to 11, wherein said first plurality of probes and said second plurality of probes are provided on separate spots of a microarray.

13. A processing unit comprising a computer program product which, when executed on a computing device, performs a method for determining the presence of a mutant of a target polynucleotide in a sample solution according to any one of claims 1 to 12, said computer program product being configured for

- receiving first hybridization intensities for a sample solution;
- receiving second hybridization intensities for a reference solution comprising said target polynucleotide;
- analyzing the logarithm of said first hybridization intensities as a function of the logarithm of said second hybridization intensities for corresponding probes and determining the presence of a mutant polynucleotide in said sample solution based thereon.

14. A device configured for performing the method for determining the presence of a mutant of a target polynucleotide in a sample solution according to any one of claims 1 to 12, comprising one or more sets of reaction vessels, feeds for reagents connected thereto and a detection unit and a processing unit comprising the computer program product according to claim 13.

**Patentansprüche**

1. Verfahren zur Bestimmung des Vorhandenseins eines mutierten Polynukleotids in einer Probenlösung, wobei sich das mutierte Polynukleotid von einem Zielpolynukleotid unterscheidet, das eine Zielsequenz in einem oder mehreren Nukleotiden der Zielsequenz umfasst, wobei das Verfahren Folgendes umfasst:

(i) Kontaktieren der Probenlösung mit einer ersten Vielzahl von Sonden und Erhalten von ersten Hybridisierungsintensitäten für jede der ersten Vielzahl von Sonden;
(ii) Kontaktieren einer Referenzlösung, die das Zielpolynukleotid umfasst und im Wesentlichen frei von dem mutierten Polynukleotid ist, mit einer zweiten Vielzahl von Sonden und Erhalten von zweiten Hybridisierungsintensitäten für jede der zweiten Vielzahl von Sonden;

und

(iii) Analysieren des Logarithmus der ersten Hybridisierungsintensitäten als Funktion des Logarithmus der zweiten Hybridisierungsintensitäten für entsprechende Sonden und Bestimmen des Vorhandenseins des mutierten Polynukleotids basierend darauf;

wobei die erste Vielzahl von Sonden mit der zweiten Vielzahl von Sonden identisch ist und wobei die verschiedenen Sonden der Vielzahl von Sonden durch eine variierende Komplementarität zu der Zielsequenz gekennzeichnet sind.

2. Verfahren nach Anspruch 1, wobei die erste und die zweite Vielzahl von Sonden jeweils Folgendes umfassen:

- eine perfekt passende Sonde für die Zielsequenz; und
- eine Vielzahl von Sonden mit einem oder zwei nichtkomplementären Nukleotiden in Bezug auf die Zielsequenz;

wobei jede der perfekt passenden Sonden und jede der Vielzahl von Sonden an separaten Stellen auf einer Oberfläche vorgesehen sind.

3. Verfahren nach Anspruch 1 oder 2, wobei die Referenzlösung im Wesentlichen frei von Mutanten des Zielpolynukleotids ist.

4. Verfahren nach einem der Ansprüche 1 bis 3, umfassend das Bestimmen, ob zwei oder mehr parallele lineare Beziehungen zwischen Teilen des Logarithmus der ersten Hybridisierungsintensitäten als Funktion des Logarithmus der zweiten Hybridisierungsintensitäten unterschieden werden können.

5. Verfahren nach einem der Ansprüche 1 bis 4, weiter umfassend das Auswählen von Sonden der zweiten Vielzahl von Sonden, für die die Hybridisierung ein thermodynamisches Gleichgewicht erreicht hat.

6. Verfahren nach einem der Ansprüche 1 bis 5, weiter umfassend das Bestimmen der relativen Menge des Zielpolynukleotids und des mutierten Zielpolynukleotids in der Probenlösung.

7. Verfahren nach einem der Ansprüche 1 bis 5, weiter umfassend das Bestimmen, welches von einer Vielzahl von möglich mutanten Polynukleotiden in der Probenlösung vorhanden ist.

8. Verfahren nach einem der Ansprüche 1 bis 7, wobei die Probenlösung hergestellt wird durch:

- Extraktion von DNA aus einer interessierenden Probe;
- Amplifikation eines Zielpolynukleotids und Mutanten davon, die in der DNA enthalten sind, unter Verwendung eines Primerpaares, von dem ein Primer an seinem 5'-Ende eine Phosphatmodifikation aufweist, wodurch doppelsträngige DNA erhalten wird; und
- Verdauen der 5'-phosphatmodifizierten Stränge der doppelsträngigen DNA unter Verwendung von Lambda-Exonuklease.

9. Verfahren nach einem der Ansprüche 1 bis 8, wobei die Hybridisierungsintensitäten durch Emission einer Markierung induziert werden, die mit einem Hybrid verbunden ist, das durch Bindung des Zielpolynukleotids oder von Mutanten davon und den Sonden gebildet wird.

10. Verfahren nach Anspruch 9, wobei die Markierung eine Hybridisierungssequenz umfasst, die komplementär zu einer Sequenz in dem mutierten Polynukleotid und dem Zielpolynukleotid außerhalb der Zielsequenz ist.

11. Verfahren nach einem der Ansprüche 1 bis 10, wobei die erste und zweite Vielzahl von Sonden jeweils mindestens 100 Sonden umfassen.

12. Verfahren nach einem der Ansprüche 1 bis 11, wobei die erste Vielzahl von Sonden und die zweite Vielzahl von Sonden an separaten Stellen eines Mikroarrays vorgesehen sind.

13. Verarbeitungseinheit, umfassend ein Computerprogrammprodukt, das, wenn es auf einem Computergerät ausgeführt wird, ein Verfahren zum Bestimmen des Vorhandenseins einer Mutante eines Zielpolynukleotids in einer Probenlösung nach einem der Ansprüche 1 bis 12 durchführt, wobei das Computerprogrammprodukt konfiguriert wird zum

- Erhalten erster Hybridisierungsintensitäten für eine Probenlösung;
- Erhalten von zweiten Hybridisierungsintensitäten für eine Referenzlösung, die das Zielpolynukleotid umfasst;
- Analysieren des Logarithmus der ersten Hybridisierungsintensitäten als Funktion des Logarithmus der zweiten Hybridisierungsintensitäten für entsprechende Sonden und Bestimmen des Vorhandenseins eines mutierten Polynukleotids in der darauf Probenlösung basierend darauf.

14. Vorrichtung, die zur Durchführung des Verfahrens zur Bestimmung des Vorhandenseins eines Mutanten eines Zielpolynukleotids in einer Probenlösung nach einem der Ansprüche 1 bis 12 konfiguriert ist, umfassend einen oder mehrere Sätze von Reaktionsgefäßen, Zuführungen für damit verbundene Reagenzien und eine Detektionseinheit und eine Verarbeitungseinheit, die das Computerprogrammprodukt nach Anspruch 13 umfasst.

**Revendications**

1. Procédé visant à déterminer la présence d'un polynucléotide mutant dans une solution d'échantillon, lequel polynucléotide mutant diffère d'un polynucléotide cible comprenant une séquence cible par un ou plusieurs nucléotide(s) de cette séquence cible, lequel procédé comporte les étapes suivantes :

i) mettre ladite solution d'échantillon en contact avec un premier ensemble de sondes, et acquérir des premières intensités d'hybridation pour chaque sonde de ce premier ensemble de sondes,
ii) mettre une solution de référence, comprenant ledit polynucléotide cible et ne contenant pratiquement pas dudit polynucléotide mutant, en contact avec un deuxième ensemble de sondes, et acquérir des deuxièmes intensités d'hybridation pour chaque sonde de ce deuxième ensemble de sondes,
iii) et analyser, pour les sondes correspondantes, les logarithmes de première intensité d'hybridation en fonction des logarithmes de deuxième intensité d'hybridation, et déterminer, sur la base de ces analyses, la présence dudit polynucléotide mutant,

étant entendu que ledit premier ensemble de sondes est identique audit deuxième ensemble de sondes, et que les différentes sondes de ces ensembles de sondes se caractérisent en ce que leur complémentarité vis-à-vis de ladite séquence cible varie d'une sonde à l'autre.

2. Procédé conforme à la revendication 1, dans lequel lesdits premier et deuxième ensembles de sondes comprennent chacun :

- une sonde qui s'apparie parfaitement à ladite séquence cible,
- et diverses sondes comprenant un ou deux nucléotides non-complémentaires vis-à-vis de ladite séquence cible,

étant entendu que chacune desdites sondes à appariement parfait et chaque sonde desdits ensembles de sondes sont disposées en des points distincts sur une surface.

3. Procédé conforme à la revendication 1 ou 2, dans lequel ladite solution de référence ne contient pratiquement aucun mutant dudit polynucléotide cible.

4. Procédé conforme à l'une des revendications 1 à 3, comportant le fait de déterminer si l'on peut distinguer des relations linéaires parallèles, au nombre de deux ou plus, entre des parties desdits logarithmes de première intensité d'hybridation en fonction desdits logarithmes de deuxième intensité d'hybridation.

5. Procédé conforme à l'une des revendications 1 à 4, comportant en outre le fait de sélectionner les sondes dudit deuxième ensemble de sondes pour lesquelles l'hybridation a atteint l'équilibre thermodynamique.

6. Procédé conforme à l'une des revendications 1 à 5, comportant en outre le fait de déterminer les quantités relatives dudit polynucléotide cible et dudit polynucléotide cible muté dans ladite solution d'échantillon.

7. Procédé conforme à l'une des revendications 1 à 5, comportant en outre le fait de déterminer lequel, parmi un ensemble de polynucléotides mutants candidats, est présent dans ladite solution d'échantillon.

8. Procédé conforme à l'une des revendications 1 à 7, pour lequel on a préparé ladite solution d'échantillon en effectuant les opérations suivantes :

    - extraction de l'ADN d'un échantillon intéressant,
    - amplification d'un polynucléotide cible et des mutants de celui-ci contenus dans ledit ADN, au moyen d'une paire d'amorces dont l'une porte une modification phosphate à son extrémité 5',
    ce qui permet d'obtenir un ADN double-brin,
    - et digestion des brins porteurs de modification phosphate en 5' de cet ADN double-brin à l'aide d'une exonucléase lambda.

9. Procédé conforme à l'une des revendications 1 à 8, dans lequel lesdites intensités d'hybridation sont induites par l'émission d'un marqueur associé à un hybride formé par liaison dudit polynucléotide cible ou de ses mutants et desdites sondes.

10. Procédé conforme à la revendication 9, dans lequel ledit marqueur comprend une séquence d'hybridation complémentaire d'une séquence présente dans ledit polynucléotide mutant et ledit polynucléotide cible, à l'extérieur de ladite séquence cible.

11. Procédé conforme à l'une des revendications 1 à 10, dans lequel lesdits premier et deuxième ensembles de sondes comprennent chacun au moins 100 sondes.

12. Procédé conforme à l'une des revendications 1 à 11, dans lequel ledit premier ensemble de sondes et ledit deuxième ensemble de sondes sont disposées en des points distincts d'un micro-réseau.

13. Unité de traitement comprenant un produit logiciel qui, quand il est exécuté sur un ordinateur, met en oeuvre un procédé visant à déterminer la présence d'un mutant d'un polynucléotide cible dans une solution d'échantillon, conforme à l'une des revendications 1 à 12, lequel produit logiciel est configuré :

    - pour recevoir des premières intensités d'hybridation concernant une solution d'échantillon,
    - pour recevoir des deuxièmes intensités d'hybridation concernant une solution de référence comprenant ledit polynucléotide cible,
    - et pour analyser, pour les sondes correspondantes, les logarithmes de première intensité d'hybridation en fonction des logarithmes de deuxième intensité d'hybridation, et déterminer, sur la base de ces analyses, la présence d'un polynucléotide mutant dans ladite solution d'échantillon.

14. Dispositif configuré pour mettre en oeuvre un procédé visant à déterminer la présence d'un mutant d'un polynucléotide cible dans une solution d'échantillon, conforme à l'une des revendications 1 à 12, et comportant un ou plusieurs jeu(x) de récipients réactionnels, des conduits d'alimentation de réactifs raccordés à ces récipients, et une unité de détection, ainsi qu'une unité de traitement comportant un produit logiciel conforme à la revendication 13.

Fig. 1

target

*wt*

*mut*

probe

MM

MM

MM

PM

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011035801 A
- EP 0995804 A
- WO 9511995 A

**Non-patent literature cited in the description**

- **HOOYBERGHS et al.** *Biosensors and Bioelectronics,* 2010, vol. 26, 1692-1694
- **HOOYBERGHS et al.** *Nucleic Acids Res.,* 2009, vol. 37, e53
- **BLOOMFIELD VA et al.** Nucleic Acids Structures, Properties and Functions. University Science Books, 2000
- **HOOYBERGHS J et al.** *Phys. Rev. E,* 2010, vol. 81, 012901
- **HADIWIKARTA WW et al.** *Nucleic Acids Res.,* 2012, vol. 40, e138